# EUROPEAN PATENT APPLICATION

(11) **EP 2 133 069 A1**
(43) Date of publication of application: **16.12.2009**
(21) Application number: 09162590.5
(22) Date of filing: 12.06.2009
(51) Int. Cl.: A61K 9/16, A61L 15/32, A61L 24/10, A61L 26/00, C08H 1/06

(54) **Process for manufacture of gelatin solutions and products thereof**

(30) Priority: 12.06.2008 US 129216 P
(71) Applicant: Lifebond, 38900 Caesarea (IL)
(72) Inventor: Attar, Ishay, 34755, Haifa (IL); Preiss-Bloom, Orahn, 30900, Zichron Yakov (IL)
(74) Representative: Messulam, Adam Clive

(57) **Abstract**

A process for the preparation of gelatin solutions which is less than a physiologically relevant temperature, which is preferably less than about 37°C, the process comprising adding one or more transition point reducing agents, such as hydrogen bond breakers, reducing agents, plasticizers, surfactants, metal ions or denaturants (denaturing agents), to the gelatin solution. The reduced temperature processing can reduce damage to thermo sensitive pharmaceuticals ingredients or vitamins, or other heat sensitive ingredients.

## Description

### FIELD OF THE INVENTION

The present invention relates to a method of manufacturing gelatin solutions, and more specifically to a process for manufacturing high concentration or high bloom gelatin solutions at a temperature no greater than a physiologically relevant temperature.

### BACKGROUND OF THE INVENTION

Gelatin is a very useful biomaterial, obtained by the thermal denaturation of collagen, which is used industrially and medically. In pharmaceutical technology, the properties of gelatin such as film formation, thermo-reversibility and adhesion are particularly important. The most important areas of application for pharmaceutical gelatin are the manufacturing of capsules and the embedding of vitamins and active drug components. The manufacturing of soft gel and hard shell capsules is a $5 billion industry that currently uses 40,000 metric tons per year of animal-derived gelatin.

Pharmaceutical and medical products are characterized by the efficacy of their active substances and the dosage forms available. Gelatin capsules are an elegant and widespread pharmaceutical dosage form, which enable drugs to be easily and safely administered, whether in liquid, paste or solid form. At the same time, pharmaceuticals in capsule form have a high degree of bioavailability. Pharmaceutical capsules enable active ingredients to be formulated with long shelf lives, protected from the effects of light, dust, humidity, and oxygen. Depending on the nature of the substance to be encapsulated, either hard- or soft capsules can be used. Soft capsules are more suitable for liquid or paste fillings, based on oil, whilst hard capsules are generally used for powdered substances.

Hard gelatin tablets, which have been found to be easier to swallow than tablets, can be filled with active ingredients(s) and excipients, in the form of powder, pellets, granulates and liquids. By means of additional coatings, the release of the active substance can be controlled and released at the precise time and location desired.

Hard capsules are made of pure gelatin, and have a water content of about 10-15 %. These capsules are generally produced with added dye. The capsules are produced using an immersion process, and subsequently supplied to the pharmaceutical industry as closed empty capsules. In a separate process, the capsules are then opened, filled with the active ingredient and any excipients, and closed.

Soft capsules on the other hand are formed, filled and closed off in a single process. Soft capsules are generally produced using the rotary die method, a process invented by Robert Pauli Scherer towards the end of the 1920s. In this process, two dyed and highly elastic bands of gelatin are passed through rollers. Whilst the capsules are being formed, they are filled with the required active ingredients and excipients. A rotary die machine can produce between 10,000 and 100,000 soft gelatine capsules, depending on their size, per hour. The capsules can be coated with appropriate substances if the active ingredients are to be released only once they have reached the intestinal tract (gastric juice-resistant).

Gelatin-coated tablets (caplets) represent a new technical development in this area. Using an immersion process, tablets are covered with a gelatin film and subsequently dried. This particular technology enables the economical advantages of tablet manufacture to be combined with the advantages of gelatin capsules.

Gelatin plays an important part in the preparation of oil-based vitamin (A+E) preparations of long shelf life and easy applicability, both for human and animal consumption. Finely distributed vitamin A or E oil drops, as well as carotinoids and polyunsaturated fatty acids, in aqueous gelatin solution can be converted, by means of appropriate solidification and drying procedures, into a free-flowing powder. This can then be dissolved in aqueous solution but remains highly dispersed. Vitamins coated with such gelatin are protected from light and oxygen during long-term storage.

The strength of the thermoreversible gels formed by a particular gelatin is expressed in terms of blooms. Higher bloom values indicate a stronger gel and represent higher molecular weights. Gelatin is commercially available in a wide range of blooms from 100 to 300.

One disadvantage of high bloom and/or high concentration gelatin solutions is that they undergo thermo-reversible gelation at temperatures of less than about 37°C. Such solutions are therefore usually prepared at temperatures of 50-60°C. Due to this attribute, it is also difficult to dissolve dry gelatin at temperatures below 37°C without granulation. Temperatures of 50-60°C may partially damage the vitamins or active pharmaceutical ingredients and make the product less effective. Furthermore, heating requires energy expenditure, reducing the cost-effectiveness of the process. The use of liquefying agents or lyotropic agents in adhesives containing relatively high concentrations of hydrolyzed collagen, i.e., about 30-50 weight percent, has been known for some time. For example, U.S. Pat. No. 1,394,654, No. 1,844,679, No. 1,873,580, No. 1,950,483, No. 2,048,499, No. 2,126,305 and No. 2,658,001 teach that thiourea, urea, biuret, alkali metal nitrates, dextrines, infusorial or diatomaceous earth, alkaline earth salicylates, ethyl alcohol, glycerol, furfuryl or tetrahydrofurfuryl alcohols, 2-methoxy methanol, thiocyanate salts and the like are useful to lower the gelling temperature of various hydrolyzed collagen-containing compositions.

U.S. Pat. No. 4,344,181, relating to a gelled gelatin food product prepared from non-gelled aqueous gelatin compositions describes another system with similar use of agents. However, all of these references teach the use of heating.

### SUMMARY OF THE INVENTION

There is a need for, and it would be useful to have, a process for the preparation of high concentration or high bloom gelatin solutions which does not require heating.

The present invention provides a process for the preparation of gelatin solutions at a temperature that is lower than a physiologically relevant temperature, which is preferably less than about 37°C, the process comprising adding one or more transition point reducing agents, such as hydrogen bond breakers, reducing agents, plasticizers, surfactants, metal ions or denaturants, to the gelatin solution. These gelatin solutions may be advantageously used for many different applications, including but not limited to the preparation of gelatin capsules. Without wishing to be limited in any way, these solutions are preferably able to support production of such capsules or coatings for capsules or tablets, or for production of other carriers of sensitive material, such as pharmaceuticals, biologics and the like. The reduced temperature also reduces potential or actual damage to such sensitive materials.

According to some embodiments of the present invention, there is provided a process for the preparation of a gelatin solution at a temperature that is lower than a physiologically relevant temperature, the process comprising adding a transition point reducing agent to the gelatin solution so as to reduce the sol-gel transition point temperature to a temperature below the transition point of the gelatin solution in the absence of the transition point reducing agent.

Preferably, the temperature is less than about 37°C.

Also preferably the transition point reducing agent comprises a hydrogen bond destroyer. Optionally and preferably the hydrogen bond destroyer comprises a reducing agent, a chaotropic agent or any other denaturing agent.

Optionally and preferably the reducing agent comprises a disulfide bond reducing agent.

Optionally and preferably the disulfide bond reducing agent comprises a thiol containing reducing agent or a phosphine-containing agent.

Optionally and preferably the thiol containing reducing agent comprises one or more of 2-mercaptoethanol, DTT, cystein, glutathione, or some combination of these agents.

Optionally and preferably the phosphine-containing agent comprises tris(2-carboxyethyl) phosphine (TCEP).

Optionally and preferably the reducing agent is selected from the group consisting of hexafluoroisopropanol (HFIP), dimethyl sulfoxide (DMSO), sodium dodecyl sulfate (SDS), glutathione, and hydroquinone.

Optionally and preferably the reducing agent comprises hydroquinone.

Optionally and preferably a concentration of the hydroquinone is in the range of from about 0.2 M to about 0.5 M.

Optionally and preferably the concentration of the hydroquinone is in the range of from about 0.3 M to about 0.4 M.

Optionally and preferably the chaotropic agent is selected from the group consisting of urea, guanidinium chloride, and lithium perchlorate.

Optionally and preferably the transition point reducing agent comprises a plasticizer.

Optionally and preferably the plasticizer is selected from the group consisting of dibutyl sebacate, polyethylene glycol and polypropylene glycol, dibutyl phthalate, diethyl phthalate, triethyl citrate, tributyl citrate, acetylated monoglyceride, acetyl tributyl citrate, triacetin, dimethyl phthalate, benzyl benzoate, ethanolamine, butyl and/or glycol esters of fatty acids, refined mineral oils, oleic acid, castor oil, corn oil, camphor and glycerol or a combination thereof.

Optionally and preferably the transition point reducing agent comprises a surfactant.

Optionally and preferably the transition point reducing agent comprises a metal ion.

Optionally and preferably the metal ion is selected from the group consisting of magnesium chloride and calcium chloride.

Optionally and preferably a concentration of the metal ion in the solution is in the range of from about 1.5 M to about 2.5 M.

Optionally and preferably the concentration is in the range of from about 1.8 M to about 2.2 M.

Optionally and preferably the transition point reducing agent comprises a denaturant.

Optionally and preferably the denaturant is selected from the group consisting of urea, a detergent, ethanol, acetone, a strong acid, a strong alkali, chemical dextrose and guanidine hydrochloride.

Optionally and preferably the denaturant comprises urea.

Optionally and preferably a concentration of the urea is in the range of from about 3M to about 5M, wherein a concentration of the gelatin solution is greater than about 20% w/w, and wherein the a bloom of the gelatin is greater than about 250.

Optionally and preferably a concentration of the urea is in the range of from about 1.5M to about 3.5M, wherein a concentration of the gelatin solution is greater than about 10% w/w, and wherein the a bloom of the gelatin is greater than about 250.

Optionally and preferably a concentration of the urea is in the range of from about 1.5M to about 3.5M, wherein a concentration of the gelatin solution is greater than about 20% w/w, and wherein the a bloom of the gelatin is greater than about 175.

Optionally and preferably a concentration ratio of the urea is in the range of from about 0.5:1 to about 1:1 urea:gelatin, weight per weight.

Optionally and preferably the denaturant comprises guanidine hydrochloride.

Optionally and preferably a concentration of the guanidine hydrochloride is in the range of from about 1:2 to about 2:2 guanidine hydrochloride: gelatin, weight per weight.

According to some embodiments, the process further comprises adding at least two transition point reducing agents selected from the group consisting of hydrogen bond breakers, reducing agents, plasticizers, surfactants, metal ions, and denaturants, to the gelatin solution.

Optionally and preferably the at least two transition point reducing agents provide a synergistic effect.

Optionally and preferably the at least two transition point lowering agents comprise calcium chloride and urea. Optionally and preferably a concentration of the urea is about 2 M and a concentration of the calcium chloride is about 1 M. Optionally and preferably a concentration of the urea is about 3 M and a concentration of the calcium chloride is about 0.5 M.

Optionally and preferably the gelatin solution is high concentration or high bloom gelatin solution. Optionally and preferably a concentration of the high concentration gelatin solution is in the range of from about 15% to about 40% w/w of total solution.

Optionally and preferably a bloom value of the high bloom gelatin solution is in the range of from about 200 to about 300.

According to some embodiments, there is provided a coating for a capsule or tablet, the coating comprising at least one cross-linked gelatin layer, wherein the crosslinking is accomplished by a non-toxic crosslinker, prepared as described herein.

According to some embodiments of the present invention, there is provided a matrix for drug delivery, the coating comprising at least one cross-linked gelatin layer, wherein the crosslinking is accomplished by a non-toxic crosslinker, prepared as described herein.

Optionally and preferably, the hydrogen bond breaker is selected from the group consisting of a plasticizer, surfactant and a denaturant, or mixtures thereof.

According to some embodiments of the present invention, there is provided a matrix comprising at least one cross-linked gelatin layer, in which one or more substances are physically suspended within the cross-linked gelatin layer. Such a matrix may optionally be used for delivery of a therapeutic substance, including but not limited to any type of drug, protein, peptide, antibody, nucleotide based agent (such as DNA or RNA for example) and so forth.

According to a further aspect of the present invention, there is provided a coating for a capsule or tablet, the coating comprising at least one cross-linked gelatin layer, wherein the crosslinking is accomplished by a non-toxic crosslinker. The non-toxic crosslinker may be an enzymatic crosslinker, such as, for example, transglutaminase.

According to a further aspect of the present invention, there is provided a coating for a capsule or tablet, the coating comprising at least one layer comprising gelatin and a transition point reducing agent.

According to a further aspect of the invention, the present inventors surprisingly found that the standard gelatin coating for soft tablets will not be necessary if one or more of the suggested compositions of the present invention for low transition point gelatin is applied instead. When the low transition point gelatin is applied with a non-toxic method for crosslinking, it will replace the coating and retard the release of the active substance as desired. Since the entire process is performed at room temperature or slightly above, there is also minimal damage to the active components of the capsule. The crosslinking can optionally be partial, depending on the required release profile. It can optionally be done by an enzyme such as transglutaminase.

As used herein, "about" means plus or minus approximately ten percent of the indicated value.

Other features and advantages of the invention will be apparent from the following detailed description, and from the claims.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in order to provide what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

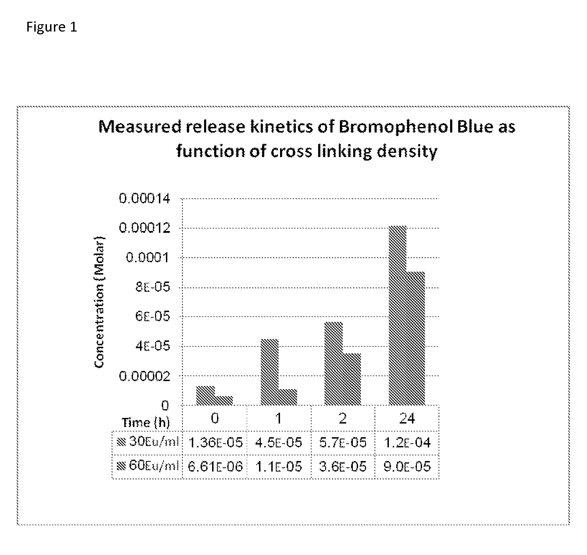

Figure 1 shows the results for the Bromophenol Blue concentrations released from the gels as function of time.

### DESCRIPTION OF THE PREFERRED EMBODIMENTS

The present invention is of a process for the preparation of gelatin solutions at a temperature that is lower than a physiologically relevant temperature, which is preferably less than about 37°C, by adding transition point reducing agents, such as hydrogen bond breakers, reducing agents, plasticizers, surfactants, metal ions or denaturants (denaturing agents), to the gelatin solution. As previously noted, the gelatin may then optionally and preferably be used to produce capsules or coatings for capsules or tablets, or for production of other types of carriers, which may then preferably be used for sensitive materials, including but not limited to pharmaceuticals and biologics.

The preparation of high-concentration gelatin solutions (15-40%) is much more complicated than that of low-concentration gelatin solutions. Fine-particle gelatin cannot be used for the preparation of highly concentrated gelatin solutions, since the particles take up water too quickly, so that too little remains for the gelatin added at the end of the process. In addition, lumps can be formed that can be difficult to dissolve. Furthermore, long stirring is needed, which creates problematic accumulation of air bubbles in the gel.

In a typical solution in the art, the gel solution is therefore warmed to a temperature of around 50-60°C to dissolve the gelatin smoothly. To prevent the formation of air bubbles, the solution is not stirred until a large proportion of the gelatin has gone into solution. Should air bubbles be formed, the solution may optionally be degassed by allowing it to stand for a longer period at 60°C.

Heating keeps quality losses at a minimum. However, heating requires the use of costly energy, thereby reducing the cost-effectiveness of the process; furthermore, heating is detrimental to the use of the resultant solution under physiological conditions (for example, for treatment of a tissue of a subject).

The present inventors have surprisingly found that addition of one or more transition point reducing agents, such as hydrogen bond breakers, reducing agents, plasticizers, surfactants, metal ions or denaturants (denaturing agents) to a high concentration and/or high bloom gelatin solution, lowers the sol-gel transition point temperature to any desired temperature below the transition point of natural gelatin, which is about 37°C. Therefore the resultant solution may optionally be used under physiological conditions at a physiological temperature, or even at a lower temperature.

Without wishing to be limited to a single hypothesis or to a closed list of effects, the present invention provides an economical and efficient process for preparation of high concentration gelatin solutions without the need for heating, by lowering the sol-gel transition point of gelatin and thereby reducing the amount of time and energy need to dissolve and/or degas the gelatin.

Furthermore, in at least some embodiments, the present invention provides a product potentially having greater activity of active pharmaceutical ingredients or vitamins, or indeed of any other temperature sensitive ingredient(s).

The process of the present invention also makes dry gelatin easier to dissolve.

According to some embodiments of the present invention, there is provided a process for preparing gelatin solution at a temperature that is lower than a physiologically relevant temperature, which is preferably less than about 37°C, the process comprising adding one or more transition point reducing agents, such as hydrogen bond breakers, reducing agents, plasticizers, surfactants, metal ions or denaturants, to the gelatin solution.

The transition point temperature depends on the particular transition point reducing agent used, and on the ratio of gelatin to the transition point reducing agent. For example, a 1:1 gelatin:urea ratio reduces the transition point to about 22°-24°C. In addition and without limitation, the effect of one or more other ingredients or components of the solution may also optionally impact upon the agent (or combination thereof) selected and/or the ratio of gelatin to the transition point reducing agent employed.

Suitable denaturants include, for example and without limitation, urea, urea nitrate, thiourea, a detergent, ethanol, acetone, a strong acid, a strong alkali, chemical dextrose, and guanidine hydrochloride (GuHCl).

Optionally and preferably, the denaturant is urea because it is a physiologically present molecule. The use of urea as a hydrogen bond destroyer causes partial denaturation of the gelatin, and will not significantly inhibit the non-thermorevesible crosslinking of the gelatin. Urea has previously been used as a food ingredient, together with gelatin, as described in U.S. Patent No. 4,341,810.

A preferred concentration ratio range for urea use is from about 2M to about 5M in gelatin solutions above a concentration of 20% w/w using gelatin above 250 bloom. For gelatin solutions above a concentration of 10% w/w with gelatin above 250 bloom or above a concentration of 20% w/w with gelatin above 175 bloom, a preferred concentration ratio range for urea is from about 1.5M to about 3.5M.

Optionally and preferably, a concentration ratio for urea is in the range of from about 0.5:1 to about 1:1 urea: gelatin, weight per weight.

Alternatively, in embodiments wherein the denaturant comprises GuHCl, a concentration ratio for GuHCl is in the range of from about 1:2 to about 2:2.

Non-limiting examples of suitable plasticizers include dibutyl sebacate, polyethylene glycol and polypropylene glycol, dibutyl phthalate, diethyl phthalate, triethyl citrate, tributyl citrate, acetylated monoglyceride, acetyl tributyl citrate, triacetin, dimethyl phthalate, benzyl benzoate, ethanolamine, butyl and/or glycol esters of fatty acids, refined mineral oils, oleic acid, castor oil, corn oil, camphor and glycerol or a combination thereof.

A reducing agent can optionally and preferably be selected from the group consisting of hexafluoroisopropanol (HFIP), dimethyl sulfoxide (DMSO), sodium dodecyl sulfate (SDS), glutathione, and hydroquinone.

More preferably, the reducing agent is hydroquinone. Optionally and preferably, hydroquinone is present in solution of the mixture at a concentration of from about 0.2 M to about 0.5 M. Most preferably, the concentration is from about 0.3 M to about 0.4 M.

A metal ion can optionally and preferably be selected from the group consisting of magnesium choride and calcium chloride. Preferably, the metal ion is present in solution of the mixture at a concentration of from about 1.5 M to about 2.5 M. More preferably the concentration is from about 1.8 M to about 2.2 M.

### Suitable denaturants include, for example, urea, a detergent, ethanol, acetone, a strong acid, a strong alkali, chemical dextrose and guanidine hydrochloride

### Optionally, a gelatin hydrolysate composition that does not undergo thermoreversible gelation is used. An example of such a composition is described in PCT WO/2006/128685, which describes a process for making a low molecular weight gelatin hydrolysate and gelatin hydrolysate compositions, wherein the low molecular weight hydrolysates create an effect that inhibits crosslinking.

In another embodiment of the current invention, two or more of the above methods of disrupting the thermo-reversible gelation of gelatin are used together. Though thermoreversible gelation can be prevented by the addition of any one of the above transition point reducing agents, comprising plasticizers, denaturants, reducing agents, and metal ions, in many cases, synergy can be achieved wherein smaller amount of two agents can be used to achieve the same effect as larger amount of a single agent.

In a preferred embodiment of synergy between transition point-lowering agents, calcium chloride and urea are both added to a gelatin solution. Preferably, 2 M of urea with 1 M of calcium chloride or 3 M of urea with 0.5 M of calcium chloride can be added to a gelatin solution above 20% w/w with gelatin above 250 bloom to keep it in liquid form. An example of this effect is described in Example 2.

The gelatin solution of the present invention may be used in any application for which gelatin is normally used. For example, hard or soft capsules may be coated with a non-thermo reversible layer, which is cross-linked by a biocompatible cross-linker, wherein one such cross-linker can be an enzyme, such as transglutaminase or microbial transglutaminase. The layer can be the sole layer or as adjunct to other layer and create a longer lasting capsule, to release the active substance as desired. The processing of such coating can incorporate a gelatin solution produced at a temperature that is lower than a physiologically relevant temperature, which is preferably less than about 37°C, for more efficient processing and reduced damage to the active component which is usually thermo-sensitive.

Controlled release of the active substance can be achieved by coating the capsules and by partial cross-linking of the low gelation point gelatin. Partial cross-linking can be achieved by halting the enzymatic reaction, either by physical or chemical inhibition of the enzyme.

If the transition point changing substance is preferably removed after the process, it may optionally be removed by applying an additional neutralizing substance including but not limiting urease enzyme for removing the urea or calcium sequestering agent for removal of the calcium; and so forth.

An emulsion comprising gelatin, sugar (or another suitable excipients) and an active ingredient to be encapsulated may be spray-dried and cross-linked, depending on the desired drug release profile. Examples of this technology are vitamin preparations, which may optionally be single vitamin preparations or preparations featuring combinations of vitamins, effervescent tablets and various beverages. The conversion of the oily substances in capsules means that they can be compressed into tablets with excellent drug release profiles. The crosslinking can be made with a non-toxic crosslinker which is preferably an enzyme, more preferably transglutaminase.

Non-limiting examples of such vitamins include any type of fat-soluble nutrients including but not limited to mono-, di- or tri-glycerides having at least one mono- or polyunsaturated fatty acid chain, as well as the fat-soluble vitamins A, D, E and K. Certain of the fat-soluble glyceride nutrients are often referred to as essential fatty acids (EFAs), including but not limited to the omega-3 and omega-6 categories of fatty acids.

In the manufacturing of sugar-coated or other types of coated tablets, the tablet core containing the active substance is coated with a thin layer of gelatin. This ensures a better binding of the sugar coating or other coating on the gelatin-coated core.

In wet granulation, a medium-Bloom aqueous solution of hide split, bone or pigskin gelatine is used as binding agent.

In the manufacture of zinc paste dressings, high-Bloom hide split and bone gelatin is applied.

Rectal and vaginal suppositories based on gelatine/glycerol are frequently used. To achieve the required melting temperature, Bloom values of 150-250 are necessary (requirement of the European Pharmacopoeia). The inventors have surprisingly found that a low gelation point gelatin can be used and can be crosslinked by a non-toxic crosslinker such as transglutaminase to meet the desired release profile of the active components to the body. The coating can be designed in such a way that the release of active substance can be controlled.

### EXAMPLES

Reference is now made to the following examples, which together with the above description, illustrate the invention in a non limiting fashion.

### Example 1: Effect of urea on gelation and cross linking of gelatin

This Example relates to the effect of urea as part of an exemplary composition according to the present invention. Urea was found to lower the transition point of gelatin solutions. The below data confirm that it sufficiently lowers the transition point of even high concentration gelatin solution to below that of room temperature so that the solution is still in liquid form at room temperature. Transglutaminase was found to be able to cross-link gelatin even in the presence of urea.

### Gelatin solution preparation:

Type A 300 bloom porcine gelatin (Sigma, St. Louis, MO) was used. 25% and 15% w/w gelatin solutions were prepared by dissolving 50 gr and 30 gr of gelatin in 150 ml and 170 ml of Dulbecco's PBS (Biological Industries, Israel), respectively, while stirring on a hot plate at 50°C. Gelatin was added to PBS gradually and stirred manually using a glass rod. Gelatin solutions were kept in a water bath at 50°C over the course of the experiment.

### Transglutaminase solution preparation:

Activa TI-WM (Ajinomoto, Japan) microbial transglutaminase (mTG) mixture was dissolved in Dulbecco's PBS to form a 20% w/w solution. The solution was maintained at room temperature (RT) over the course of the experiment.

### Gelatin-urea solution preparation:

40 g aliquots of 25% or 15% w/w gelatin solutions were transferred to 100 ml beakers and stirred at 50°C.

Urea (98%, Alfa Aesar, Lancaster, UK) was added to these beakers in different ratios, as detailed in the below table:

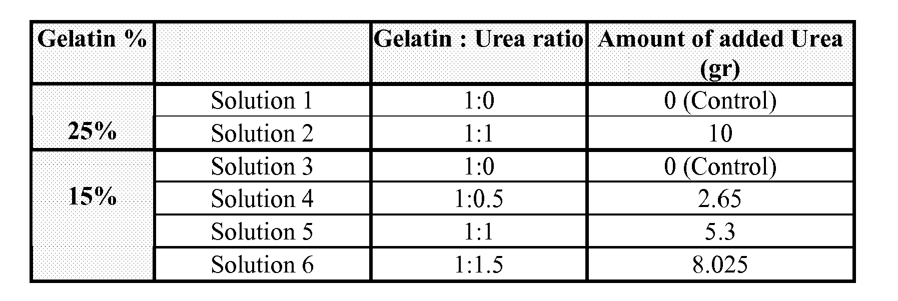

After urea addition, the gelatin-urea solution was stirred at 50°C for 5 min to ensure homogeneity and then transferred to a 37°C water bath.

Each gelatin-urea solution was removed, in turn, from the 37°C water bath and left to cool at RT. A thermometer was used to follow the temperature decrease of each solution. The appearance and viscosity of the gelatin-urea solution were assessed as the temperature decreased by observation and palpating the solution with a glass rod.

Results are summarized in Table 1.

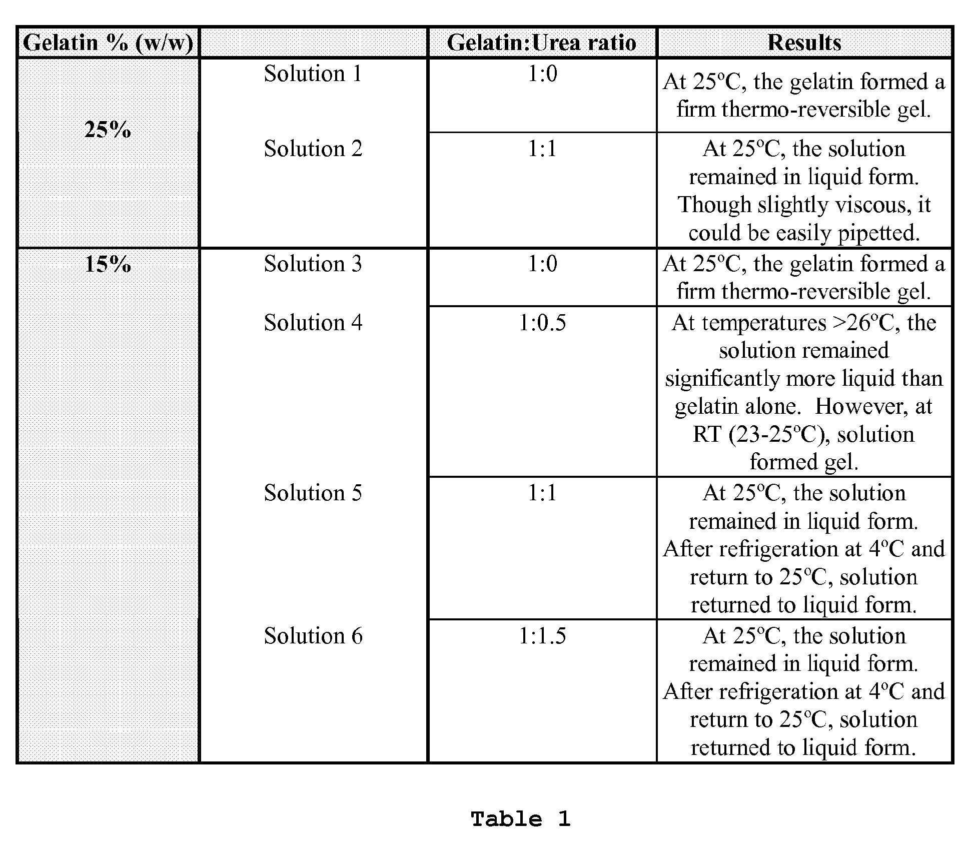

### Cross linking of gelatin-urea solution using mTG

Gelatin-urea solutions were cross linked using mTG. 1 or 2 ml of 20% w/w mTG solution was manually mixed with 2 ml of gelatin-urea solution in a plastic dish. The gelatin-urea solution was added either at RT or preheated to 50 °C. In some tests, it was heated to 50 °C to facilitate comparison with gelatin alone, which needs to be heated to be mixed with mTG. The solutions were mixed by gently stirring with the pipette tip and left to cross-link for several minutes. As in examination of thermo-reversible gel formation, the appearance and viscosity of the gelatin-urea solution after mixture with mTG were assessed by observation and by palpating the solution with a glass rod.

The results are summarized in Table 2 on the following page.

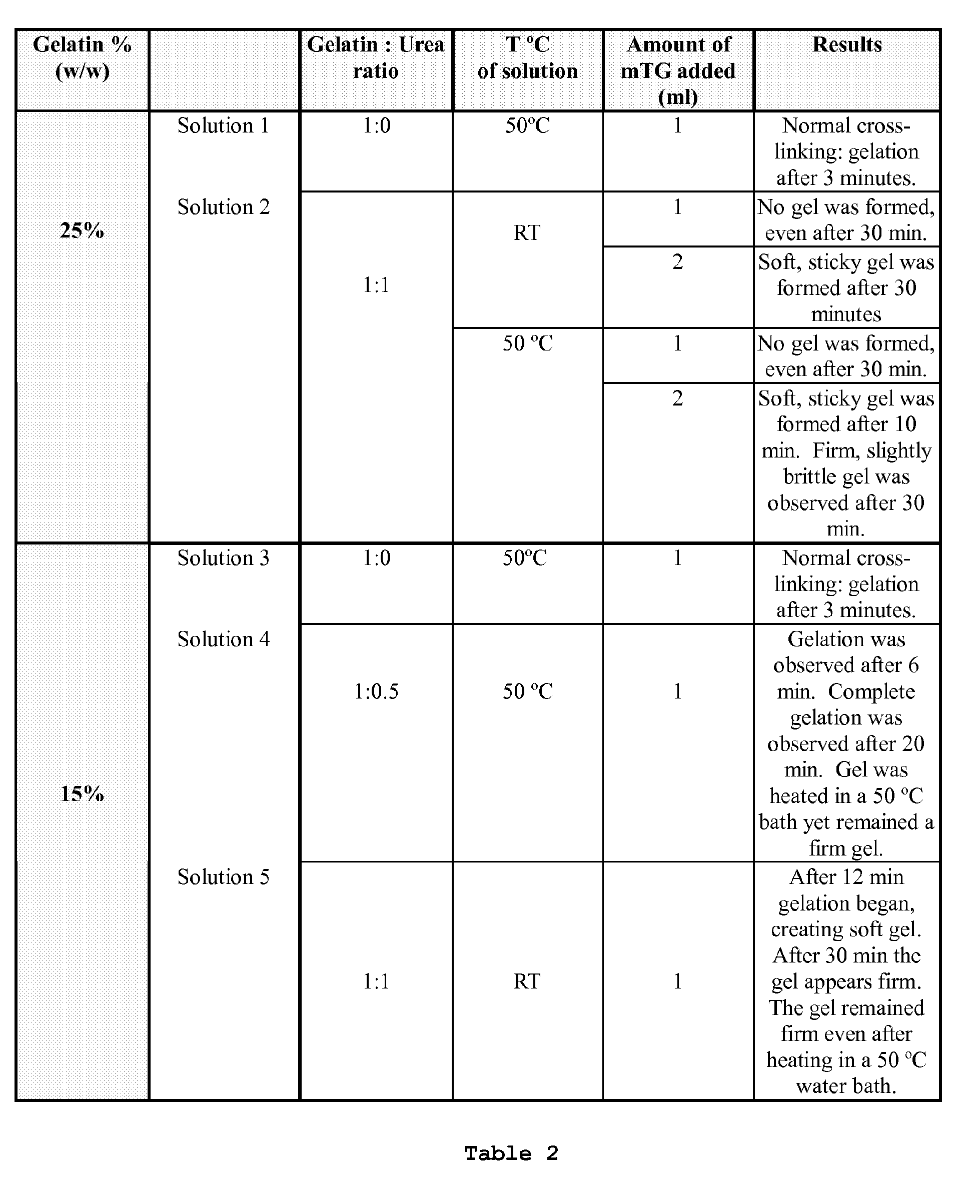

The above studies show that addition of urea to gelatin solutions decreases the transition point of gelatin significantly. For both 15% and 25% w/w gelatin solutions, the transition point is reduced to below RT by the addition of urea at ratios of 1:1 urea:gelatin and above. At urea:gelatin ratios of 0.5:1, the transition point of the gelatin solutions is slightly above RT. It is likely that a urea:gelatin ratio between 0.5:1 and 1:1 will suffice to lower the transition point of gelatin below RT.

It was also shown that cross-linking of gelatin using mTG in the presence of urea is possible. However, urea has a detrimental effect on mTG activity. It appears that this effect is relative to urea concentration, such that mTG activity in the presence of urea is inversely proportional to the urea concentration.

Transglutaminase activity at 50°C was far greater than mTG activity at RT, as would be expected. The addition of urea to gelatin can be optimized to find the minimum concentration that reduces the gelatin transition point below RT. If a sufficient amount of mTG is added, it should be able to overcome the detrimental effect of urea.

### Example 2: Synergistic effect of urea & calcium chloride on thermo-reversible gelation of gelatin:

### Materials

The following materials were used in the experiments: 300 bloom type A porcine gelatin [Sigma, St. Louis, Missouri], 98% urea [Alfa Aesar, Lancester], Calcium Chloride 97% [Alfa Aesar, Lancester], PBS - Dulbecco's Phosphate Buffered Saline without Calcium and Magnesium [Biological Industries, Israel], Microbial Transglutaminase ACTIVA- WM, 1 % enzyme powder in maltodextrin [Ajinomoto, Japan].

### Methods

Stock solutions of 5 M calcium solution, 5 M of urea solution were prepared. 25% (w/w) gelatin solution, urea and calcium solutions were prepared by diluting urea and calcium stock solutions.

### Control A- gelatin was dissolved in PBS

Control Calcium A- gelatin was dissolved in 2 M Calcium.
Control Calcium B - gelatin was dissolved in 1 M calcium.
Solution 1- gelatin was dissolved in PBS solution containing 1 M calcium and 2 M urea.
Solution 2- gelatin was dissolved in PBS solution containing 1 M calcium and 3 M urea.
Solution 3- gelatin was dissolved in PBS solution containing 0.5 M calcium and 2 M Solution 4- gelatin was dissolved in PBS solution containing 0.5 M calcium and 3 M urea.
Solution 5- gelatin was dissolved in PBS solution containing 4 M urea.

### Results

Table 3 summarizes the experimental results for gelatin gels cross linked (XL) with mTG:

**Table 3**

| Gelatin Solution | Additives | State at 24°C | Description |
|---|---|---|---|
| Control | - | Gelled | Clear gel |
| Control Calcium A | 2 M Ca | Liquid | Opaque solution |
| Control Calcium B | 1 M Ca | Highly viscous | Opaque gel |
| Solution 1 | 1 M Ca 2M urea | Liquid | Opaque solution |
| Solution 2 | 1 M Ca 3 M Urea | Liquid | Opaque solution |
| Solution 3 | 0.5 M Ca 2 M urea | Highly viscous | Opaque solution |
| Solution 4 | 0.5 M Ca 3 M Urea | Viscous | Opaque soluution |

As shown in Table 3, urea and calcium have a synergistic effect on reducing the transition point of 25% (w/w) gelatin solutions. 25% (w/w) gelatin solution containing 1 M Calcium chloride combined with 2 M of Urea has a low viscosity at RT. 25% (w/w) gelatin solution containing 0.5 M Calcium chloride and 3 M urea is viscous at RT.

### Example 3

### Controlled Drug Delivery

This example provides an initial in vitro demonstration of a gel matrix made of an enzymatically crossed linked protein that serves as a drug delivery system with a controlled release.

### Materials:

A gelatin component containing 25% (w/w) gelatin (porcine, type A, 275 bloom) dissolved in a 0.1M Na-Ac (Sigma-Aldrich, St. Louis) buffer pH 6.0 at 37°C with 3.8 M urea (Sigma- Aldrich, St. Louis) and 0.15M CaCl2 (Sigma- Aldrich, St. Louis) was prepared. Food grade microbial Transglutaminase enzyme (Activa in maltodextrin WM) was obtained from Ajinomoto^{™}. 0.2M Na-Citrate (Sigma- Aldrich, St. Louis) buffer pH 6.0 was prepared. Bromophenol Blue (Mw= 691.9) was purchased from Bio-Rad Laboratories (CA). Dulbecco's Phosphate Buffered Saline (PBS) was obtained from Biological Industries (Kibbutz Beit HaEmek).

### Procedure:

Enzyme components containing 30 EU/mL and 90 EU/mL were prepared by dissolving microbial transglutaminase in Na-Citrate buffer containing 0.5% (w/v) Bromophenol Blue. 0.66 mL of each enzyme component was mixed with 1.33 mL of the gelatin component to yield a crossed linked gel matrix. The mixed solutions were immediately cast to moulds to form triplicates of 1.7 mm thick films and left to cure for 9 minutes. The final Bromophenol blue concentration was 4.93mM. After 9 minutes each film was submerged in 20 mL of PBS solution and incubated at 37°C with orbital shaking. Bromophenol Blue concentrations in the extracts was measured at t=0, 1h, 2h and 24h. The optical density of the extract solutions was measured using a spectrophotometer at the wavelength of 592nm. The concentration was determined using the molar extinction coefficient of Bromophenol Blue (ε592nm=78000).

To examine the effect of the cross linking density on the drug release kinetics from the gel matrix, the release from gels prepared with 30 EU/mL microbial transglutaminase components were compared to the kinetics of gels prepared with 60 EU/mL microbial transglutaminase components.

### Results:

Figure 1 shows the results for the Bromophenol Blue concentrations released from the gels as function of time. The blue bars shows the measured concentrations from gels crossed linked with enzyme component containing 30 EU/mL and the red bars indicate the concentrations released from gels crossed linked with enzyme component containing 60 EU/mL.

The results show that Bromophenol Blue is released to the matrix in a controlled manner, as indicated by the increase of its concentration in the extract solution as function of time.

The results also indicate that the amount of cross linker in the gel affects the release of Bromophenol Blue from the gel matrix. The higher the enzyme concentration is, the lower the released Bromophenol Blue concentration.

The results indicate that the suggested gel matrix can serve as a drug delivery system. Controlled release of small molecules such as Bromophenol Blue (Mw= 691.9) is feasible. The release profile can be tailored through controlling the cross linking density of the gel matrix.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A process for the preparation of a gelatin solution at a temperature that is lower than a physiologically relevant temperature, the process comprising adding a transition point reducing agent to the gelatin solution so as to reduce the sol-gel transition point temperature to a temperature below the transition point of the gelatin solution in the absence of said transition point reducing agent.

2. The process of claim 1, wherein said temperature is less than about 37°C.

3. The process of claims 1 or 2, wherein said transition point reducing agent comprises one or more of a hydrogen bond destroyer, a disulfide bond reducing agent, a plasticizer, a surfactant, a metal ion, guanidine hydrochloride, or optionally comprises at least two of these agents.

4. The process of claim 3, wherein said hydrogen bond destroyer comprises a reducing agent, a chaotropic agent or any other denaturant; and/or wherein said disulfide bond reducing agent comprises a thiol containing reducing agent or a phosphine-containing agent; and/or wherein said plasticizer is selected from the group consisting of dibutyl sebacate, polyethylene glycol and polypropylene glycol, dibutyl phthalate, diethyl phthalate, triethyl citrate, tributyl citrate, acetylated monoglyceride, acetyl tributyl citrate, triacetin, dimethyl phthalate, benzyl benzoate, ethanolamine, butyl and/or glycol esters of fatty acids, refined mineral oils, oleic acid, castor oil, corn oil, camphor and glycerol or a combination thereof; and/or wherein said metal ion is selected from the group consisting of magnesium chloride and calcium chloride.

5. The process of claim 4, wherein said thiol containing reducing agent comprises one or more of 2-mercaptoethanol, DTT, cystein, glutathione, or some combination of these agents, or wherein said phosphine-containing agent comprises tris(2-carboxyethyl) phosphine (TCEP); and/or wherein said reducing agent is selected from the group consisting of hexafluoroisopropanol (HFIP), dimethyl sulfoxide (DMSO), sodium dodecyl sulfate (SDS), glutathione, and hydroquinone; and/or wherein said chaotropic agent is selected from the group consisting of urea, guanidinium chloride, and lithium perchlorate.

6. The process of any of claims 3-5, wherein said reducing agent comprises hydroquinone and a concentration of said hydroquinone is in the range of from about 0.2 M to about 0.5 M, and is optionally in the range of from about 0.3 M to about 0.4 M.

7. The process of any of claims 3-6 wherein a concentration of said metal ion in said solution is in the range of from about 1.5 M to about 2.5 M and optionally in the range of from about 1.8 M to about 2.2 M.

8. The process of any of claims 3-7, wherein said denaturant is selected from the group consisting of urea, a detergent, ethanol, acetone, a strong acid, a strong alkali, chemical dextrose and guanidine hydrochloride.

9. The process of claim 8, wherein said denaturant comprises urea, with a concentration in the range of from about 3M to about 5M, wherein a concentration of said gelatin solution is greater than about 20% w/w, and wherein said a bloom of said gelatin is greater than about 250.

10. The process of claim 9, wherein a concentration of said urea is in the range of from about 1.5M to about 3.5M, wherein a concentration of said gelatin solution is greater than about 10% w/w, and wherein said a bloom of said gelatin is greater than about 250; or wherein a concentration of said gelatin solution is greater than about 20% w/w, and wherein said a bloom of said gelatin is greater than about 175.

11. The process of any of claims 3-10, wherein a concentration ratio of said urea is in the range of from about 0.5:1 to about 1:1 urea:gelatin, weight per weight.

12. The process of any of claims 3-11, wherein a concentration of said guanidine hydrochloride is in the range of from about 1:2 to about 2:2 guanidine hydrochloride: gelatin, weight per weight.

13. The process of any of claims 1 to 12, wherein said gelatin solution is a high concentration solution, with a concentration of said high concentration gelatin solution in the range of from about 15% to about 40% w/w of total solution; or a high bloom gelatin solution, wherein a bloom value of said high bloom gelatin solution is in the range of from about 200 to about 300.

14. A coating for a capsule or tablet, said coating comprising at least one cross-linked gelatin layer, wherein the crosslinking is accomplished by a non-toxic crosslinker, prepared according to any of claims 1-13.

15. A matrix for drug delivery, said coating comprising at least one cross-linked gelatin layer, wherein the crosslinking is accomplished by a non-toxic crosslinker, prepared according to any of claims 1-13.
